# EUROPEAN PATENT SPECIFICATION

(11) **EP 4 419 225 B1**
(45) Date of publication and mention of the grant of the patent: **17.06.2026**
(21) Application number: 22802961.7
(22) Date of filing: 17.10.2022
(51) Int. Cl.: B01D 3/00, B01D 3/14, B01D 3/42

(54) **PROCESS FOR PURIFICATION OF N-BUTANOL**
VERFAHREN ZUR REINIGUNG VON N-BUTANOL
PROCÉDÉ DE PURIFICATION DU N-BUTANOL

(30) Priority: 22.10.2021 EP 21204088
(43) Date of publication of application: 28.08.2024
(73) Proprietor: BASF SE, 67056 Ludwigshafen am Rhein (DE)
(72) Inventor: METZEN, Bernd, 67056 Ludwigshafen (DE); PAPP, Rainer, 67056 Ludwigshafen (DE)
(74) Representative: BASF IP Association
(86) International application number: PCT/EP2022/078770
(87) International publication number: WO 2023/066835

(56) References cited:
- EP-A1- 1 338 317
- EP-B1- 2 394 723
- US-A1- 2004 011 706
- US-A1- 2014 138 237
- US-B1- 6 551 465
- US-B2- 8 052 845

## Description

### TECHNICAL FIELD

The invention concerns a method for providing a high-purity n-butanol product stream from a crude n-butanol feed stream by fractional distillation in a dividing wall column comprising an upper section, a middle section and a lower section, the middle section comprising an inflow section and an offtake section laterally separated from each other by a dividing wall fixed in the column, the crude n-butanol feed stream being fed to the inflow section and the high-purity n-butanol product stream being withdrawn from the offtake section.

### BACKGROUND ART

Highly pure n-butanol is typically obtained from a mixture containing more than 90% by weight of n-butanol and further components such as 4-heptanone, dibutyl ether, 2-methylbutanol, 2-ethylhexanal, butyl butyrate, butyl acetal, isobutanol, n-butyraldehydes and water. This so-called "crude n-butanol stream" is typically separated in a distillation column sequence comprising a light-end column, a pure product column and a heavies column. The crude n-butanol stream is fed to the light-end column where components lighter than n-butanol and water are separated off overhead. The remaining mixture including the n-butanol is fed to the pure product column where a highly pure n-butanol stream is obtained as a side draw in the rectifying section of the column. Some lighter components are withdrawn at the top of the pure product column. Components heavier than n-butanol are separated from the bottom of that column and are fed to the heavies column for further separation. This classical process comes along with high investment and operating costs, in particular due to a high energy demand for the three distillation columns.

One possibility to reduce investment and energy costs is to integrate two or even three columns into one column that has a dividing wall in a middle section of the column. A dividing wall column generally has the following segments: an upper column region located above the dividing wall, an inflow section located on the side of the feed point and bounded laterally by a dividing wall, an offtake section located on the side of the side offtake and bounded laterally by the dividing wall and a lower column region located below the dividing wall. Compared to the arrangement of conventional distillation columns, dividing wall columns offer advantages in respect of both energy consumption and capital costs.

Document EP 2 394 723 B1 discloses a process for the purification of n-butanol from a crude n-butanol stream in a dividing wall column where all three columns of the classical sequence are integrated into one dividing wall column. The crude n-butanol stream is fed to the inflow section on one side of the dividing wall, whereas a highly pure n-butanol product stream is withdrawn from the offtake section on the other side of the dividing wall. Lighter components are withdrawn overhead and heavier components are obtained in the bottom of the dividing wall column. The integration of all three separation tasks, i.e. separation of lighter components, heavier components and highly pure n-butanol necessitates a rather high bottom temperature and large internal volumes of liquid and vapor circulating in the column. Consequently, this process also shows a high energy demand and thus operating costs. Furthermore, the high bottom temperature may foster the formation of unwanted byproducts that reduce the yield of the wanted product n-butanol. Unwanted byproducts that are light-boiling enrich in the top of the column which leads to an increase of the stream to be withdrawn from the top of the column and thus to an increase of energy needed to fulfill the specification of the n-butanol side draw.

It was an object of the invention to provide a method for preparing highly pure n-butanol from a crude n-butanol stream that shows a lower energy demand than processes known from prior art without compromising on the quality of the desired product n-butanol.

This task is solved according to the invention by a method according to claim 1. Advantageous variants of the method are presented in claims 2 to 6.

### BRIEF DESCRIPTION

Subject of the invention is a method for providing a high-purity n-butanol product stream from a crude n-butanol feed stream by fractional distillation in a dividing wall column comprising an upper section, a middle section and a lower section. The middle section includes an inflow section and an offtake section laterally separated from each other by a dividing wall fixed in the column, the crude n-butanol feed stream being fed to the inflow section and the high-purity n-butanol product stream being withdrawn from the offtake section. The liquid stream flowing out of the upper section is collected and divided into a first liquid stream and a second liquid stream, the first liquid stream being fed to the upper region of the offtake section and the second liquid stream being fed to the upper region of the inflow section, wherein the split ratio between the mass flow rate of the first liquid stream and the mass flow rate of the second liquid stream is from 2:1 to 5:1. It was found that a split ratio in this range has a double positive effect: The overall energy demand is decreased compared to the prior art, thereby maintaining the flexibility of reacting to disturbances or changing operating conditions, for example variations in the feed composition with respect to the ratio of light and heavy boiling components.

The dividing wall column may be of any type known in the art that is suitable for separating a crude n-butanol feed stream. The column may for example be equipped with trays like sieve trays or valve trays or with structured or random packings. With respect to a desired minimization of the pressure drop along the column height, structured packings are preferred.

The upper section has preferably from 15 to 40 theoretical stages, more preferably from 20 to 30 theoretical stages.

The lower section has preferably from 10 to 30 theoretical stages, more preferably from 12 to 20 theoretical stages.

The inflow section comprises two zones, an upper inflow zone between the upper section and the feed stage and a lower inflow zone between the feed stage and the lower section. The upper inflow zone has preferably from 8 to 22 theoretical stages, more preferably from 10 to 15 theoretical stages. The lower inflow zone has preferably from 10 to 30 theoretical stages, more preferably from 15 to 25 theoretical stages.

The offtake section also comprises two zones, an upper offtake zone between the upper section and the side-draw through with the product stream is withdrawn and a lower offtake zone between the side-draw and the lower section. The upper offtake zone has preferably from 10 to 30 theoretical stages, more preferably from 15 to 25 theoretical stages. The lower offtake zone has preferably from 8 to 25, more preferably from 10 to 15 theoretical stages.

According to the invention, the liquid flowing out of the upper section is collected and fed in two separate streams to the offtake section and the inflow section. Liquid collectors and distributors for distillation columns are known in the art.

Depending on its source, the crude n-butanol feed stream may have varying compositions. According to the invention, the crude n-butanol feed stream comprises more than 96 % by weight of n-butanol, less than 2 % by weight of water and less than 2 % of other organic components, for example isobutanol or butyl butyrate.

In a preferred embodiment the ratio between the cross-sectional area of the inflow section and the cross-sectional area of the offtake section is from 0.9 to 1.1, more preferably from 0.95 to 1.05. Most preferably the cross-sectional areas are equal, apart from tolerances due to manufacturing or installation of the dividing wall. A dividing wall column with equal or nearly equal cross-sectional areas of the two section on either side of the dividing wall is easy to manufacture, to install and to operate.

In a further preferred embodiment of a method according to the invention the temperature in the lower section is from 115 °C to 130 °C. The pressure in the lower section is preferably from 1.0 to 1.5 bar (abs), more preferably from 1.1 to 1.3 bar (abs). It has been found that under these conditions at the bottom of the dividing wall column the yield of the desired product n-butanol in the product side-draw is high on the one hand whereas on the other hand the energy demand for heating and cooling the liquid and vapor flows in the column are reasonable. At higher temperatures, for example as in the known prior art, there is an increasing risk that longer-chain components form that increase the temperature in the sump even more. There is also a risk at higher temperatures that high boiling components decompose into lighter boiling components which then flow upwards in the column and may have a detrimental effect on the purity of the product withdrawal in the side-draw.

A relatively low temperature in the lower section of the dividing wall column goes along with a higher percentage of n-butanol in the stream withdrawn from the bottom of the column. However, the advantages in terms of smooth operation of the column and a low energy demand are higher than the potential loss of the product n-butanol via the bottom flow. Therefore, the n-butanol product stream contains more than 99 % by weight of n-butanol, and a bottom product stream taken off from the bottom of the column contains more than 40 % by weight of n-butanol.

Depending on the source of the crude n-butanol stream and on the target use of the n-butanol produced by the method according to the invention, more or less side-components are allowed in the final product. In a preferred embodiment the crude n-butanol feed stream contains more than 300 ppm by weight of butyl butyrate, and the n-butanol product stream contains less than 100 ppm by weight of butyl butyrate. In a further preferred embodiment, the crude n-butanol feed stream contains more than 500 ppm by weight of isobutanol, and the n-butanol product stream contains less than 500 ppm by weight of isobutanol. In a further preferred embodiment, the crude n-butanol feed stream contains more than 500 ppm by weight of water, and the n-butanol product stream contains less than 500 ppm by weight of water. This can be achieved by choosing the split ratio in the range according to the invention.

The split ratio between the mass flow rate of the first liquid stream and the mass flow rate of the second liquid stream can be realized by different means. In one embodiment the split ratio is fixed by design, for example by an appropriate dimensioning of the pipes through which the first liquid stream and the second liquid stream flow or by installing respective valves or throttles. In a preferred embodiment the flow rates of the first liquid stream and the second liquid stream are measured and at least one of the two streams is controlled by an actuator to adjust the split ratio to a predefined value between 2:1 and 5:1. This embodiment has the advantage that the split ratio can be adjusted when needed.

In a further preferred embodiment the location of the draw-off of the liquid collected from the upper section and the location of the inlets to the distributors of the inflow section and the offtake section are spaced apart at a vertical distance. Preferably this distance is from 1 to 3 meters, more preferably from 1.5 to 2.5 meters. A vertical distance in that range provides a sufficient compensation of the pressure drop induced by installed equipment like flow meters, flow control valves or piping without compromising too much on installation space needed. No pumps or other means for forced transportation are necessary in that case.

It is further preferred that the pipes connecting the draw-off and the inlets are formed as u-bend, meaning that the pipe from the draw-off extends below the positions of the inlets, are deflected in a u-bend form and lead back upwards to the inlets. In such a configuration, the control instrumentation like flow meters and/or flow control valves is preferably arranged in that part of the pipes that extends below the inlets of the distributors of the inflow section and the offtake section. More preferably, the control instrumentation is arranged in that part of the pipes that is positioned between the u-bend and the inlets. Due to the u-bend form the parts of the pipes that extend below the inlets are constantly filled with liquid. An arrangement of the control instruments in that part of the pipes is advantageous as it enables consistent and proper measurements and liquid distribution control.

It is advantageous to control the quality of the high-purity n-butanol product stream. The quality can be assessed for example by analyzing the composition of the product stream. Indirect methods are also possible, for example measuring temperature and pressure near the product withdrawal and determining the product composition based thereon, e.g. using a mathematical model. There are also different options to influence the product composition, for example by appropriate settings of the reboil ratio or amount of vapor flowing up in the column or the reflux ratio or amount of condensed liquid flowing down in the column.

In a preferred embodiment the quality of the n-butanol product stream is controlled to a predefined setpoint by measuring a process value representing the quality in the product stream, in particular the amount of n-butanol in the product stream, as the controlled variable and adjusting the split ratio between the mass flow rates of the first liquid stream and the second liquid stream as the manipulated variable. It is further preferred to adjust the amount of heat provided to a reboiler, e.g. steam, as a further manipulated variable.

### DETAILED DESCRIPTION

The invention is explained in more detail below with reference to the drawings. The drawings are to be interpreted as in-principle presentation. They do not constitute any restriction of the invention, for example with regard to specific dimensions or design variants. In the figures:
- Fig. 1: shows a dividing wall column as an embodiment according to the invention.
- Fig. 2: shows a liquid distribution control system as a preferred embodiment according to the invention.

### List of reference numerals used:

- 102: Dividing wall column
- 104: Upper section
- 106: Inflow section
- 108: Offtake section
- 110: Lower section
- 112: Dividing wall
- 114: First liquid stream
- 116: Second liquid stream
- 202: Collector
- 204: First draw off nozzle
- 206: First pipe
- 208: First distributor
- 210: Second draw off nozzle
- 212: Second pipe
- 214: Second distributor
- 216: Flow meter
- 218: Flow control valve
- 220: Flow ratio controller

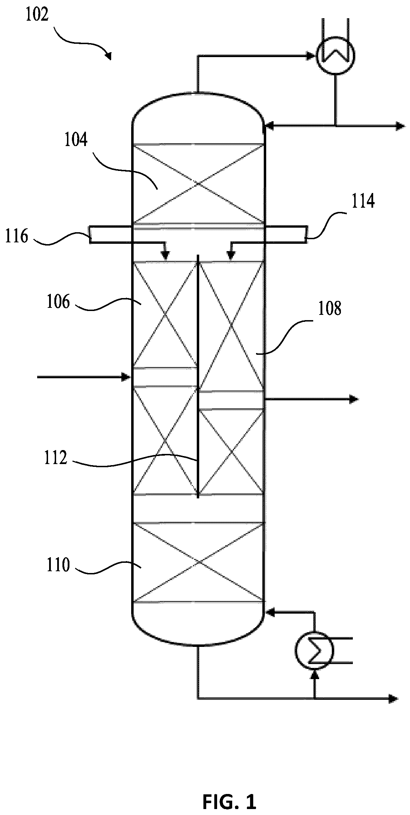

Fig. 1 shows a schematic view of a dividing wall column 102 as an embodiment according to the invention. The column comprises an upper section 104, a middle section and a lower section 110. The middle section comprises an inflow section 106 and an offtake section 108 which are separated from each other by a dividing wall 112 that is fixed in the column and extends from one inner wall to the opposite inner wall of the column. A feed stream is fed to the inflow section 106 and separates the inflow section into two zones, an upper inflow zone above the feed point and a lower inflow zone below the feed point. The offtake section 108 also comprises two zones, an upper offtake zone above the product side-draw and a lower offtake zone below the product side-draw.

Liquid containing heavier boiling components that collects in the sump of the lower section 110 of the column is withdrawn from the column and is partially fed to a reboiler where it is vaporized and fed back to the lower section 110 of the column.

The vapor outlet at the top of the column is connected to a condenser where the vapor is condensed and partially recycled to the upper section 104 of the column. The remaining part of the condensed stream is withdrawn from the column as a distillate stream. The condenser may be a total condenser that condenses the complete vapor stream or a partial condenser that only condenses a part of the vapor stream. In the latter case, a vaporous stream of non-condensed components can be withdrawn from the condenser. The condenser may further comprise a phase-separation unit wherein an aqueous phase is separated from an organic phase. In such a case, the aqueous phase is withdrawn from the condenser, whereas the organic phase is at least partially recycled to the upper section 104 of the column.

The liquid stream that flows out of the upper section 104 is collected below the upper section 104 and is withdrawn from the column in two separate streams, a first liquid stream 114 and a second liquid stream 116. Both streams are measured, for example by flow meters installed in the respective pipes. The first liquid stream 114 is fed back to the column and is distributed onto the upper region of the offtake section 108 and thus to only one side of the dividing wall 112. The second liquid stream 116 is also fed back to the column and is distributed onto the upper region of the inflow section 106 and thus to the other side of the dividing wall 112. At least one of these two streams can be set to a specified value by an actuator, for example by a control valve, such that the ratio between the mass flow rate of the first liquid stream 114 and the mass flow rate of the second liquid stream 116 can be set from 2:1 to 5:1.

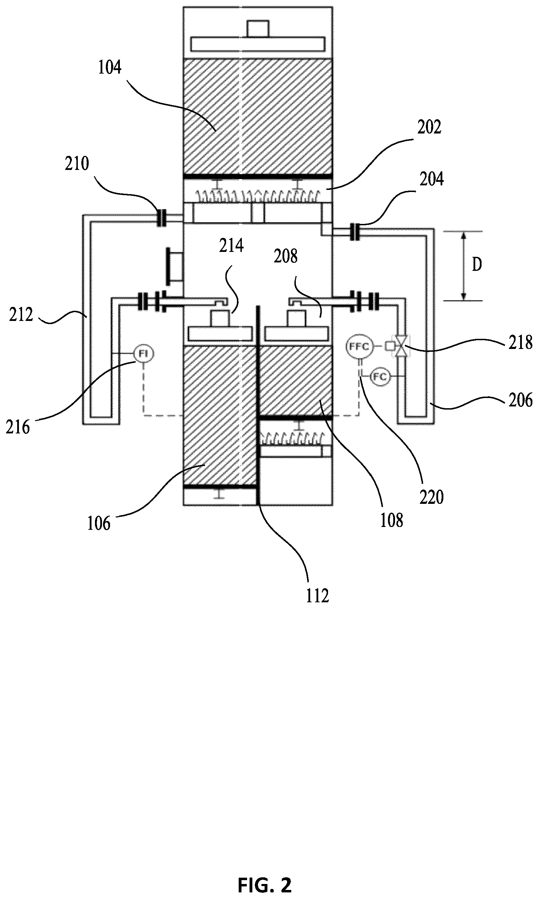

Fig. 2 shows a liquid distribution control system as a preferred embodiment according to the invention. Liquid flowing out of the upper section 104 of the dividing wall column flows into a collector 202 located below the lower end of the upper section 104. The collected liquid is withdrawn from the collector 202 in two separate streams through a first draw off nozzle 204 and a second draw off nozzle 210. Preferably, the outlets of the two draw off streams are located close to each other. This has the advantageous effect that the compositions of the first liquid stream and the second liquid stream are nearly identical and that no separation in terms of composition occurs due to a spatial distance of the draw off locations.

The first liquid stream taken off through the first draw off nozzle 204 is fed through a first pipe 206 to a first distributor 208 that is located on top of the offtake section 108 on one side of the dividing wall 112. The second liquid stream taken off through the second draw off nozzle 210 is fed through a second pipe 212 to a second distributor 214 that is located on top of the inflow section 106 on the other side of the dividing wall 112.

According to the invention, the split ratio between the mass flow rate of the first liquid stream and the mass flow rate of the second liquid stream is set to a value from 2:1 to 5:1. The example according to Fig. 2 shows a simple but yet effective solution for a control strategy to set the split ratio to a predefined value. The amount of liquid flowing through the second pipe 212 is measured by a flow meter 216. The amount of liquid flowing through the first pipe 206 is measured in a flow control valve 218. A flow ratio controller 220 compares the measured values of the amount of the first liquid stream and the second liquid stream and adjusts the flow control valve 218 such that the desired predefined value of the split ratio is realized in the dividing wall column.

Other control schemes and control systems are also possible, both in terms of instrumentation and in terms of the control logic. For example, the flow control valve 218 could also be provided in the second pipe 212 or an additional flow control valve could be provided in the second pipe.

It is preferred that the location of the draw off nozzles and the location of the inlets to the distributors are spaced apart at a vertical distance D of from 1 to 3 meters, preferably from 1.5 to 2.5 meters. A vertical distance D in that range provides a sufficient compensation of the pressure drop induced by the flow meter 216, the flow control valve 218 and piping without compromising too much on installation space needed. No pumps or other means for forced transportation are necessary in that case.

### Example 1

A laboratory-scale dividing wall column according to Fig. 1 was used to separate a high-purity n-butanol product stream from a crude n-butanol feed stream. Four single columns made of glass were connected to each other in a dividing wall configuration. The six separation zones were equipped with CY wire gauze packings from company Sulzer Chemtech. The column representing the upper section 104 had a diameter of 55 mm and the height of the packing was 1.9 m. The inflow section 106 was represented by a column with a diameter of 40 mm, the packing height of the upper inflow zone was 1.1 m and the height of the packing below the feed stage was 1.8 m. The offtake section 108 was represented by a column with a diameter of 40 mm, the packing height of the upper offtake zone was 1.6 m and the height of the packing below the product side-draw was 1.4 m. The column representing the lower section 110 was 55 mm in diameter and had a packing height of 1.0 m. The dividing wall column was operated with at a pressure of 950 mbar (abs) at the top of the upper section 104.

The crude n-butanol feed was provided to the inflow section 106 at a flow rate of 2750 g/h and a composition as given in Table 1 below (denoted as "Feed"). All compositions in Table 1 are given in area percent. The compositions were determined using a gas chromatograph without an inner standard. The gas chromatograph was equipped with a CP-Sil 19 CB column (dimensions 25 m x 250 µm x 0.2 µm). 0.2 µl were injected and the split ratio was 100:1. Detection was done with a flame ionization detector (FID) at at temperature of 300 °C.

A distillate stream containing low-boiling components was withdrawn from the condenser at a flow rate of 26 g/h. The reflux from the condenser to the top of the upper section 104 was set to 2850 g/h. A bottom sump stream of 155 g/h containing high-boiling components was withdrawn from the bottom of the lower section 110 at a temperature of about 120°C. The content of n-butanol in the sump stream was about 63 %. The side-draw containing the desired high-purity n-butanol was withdrawn at a flow rate of 2568 g/h.

The split ratio between the mass flow rate of the first liquid stream and the mass flow rate of the second liquid stream has been varied between 2:1, 3:1 and 5:1. The results depending on the different operating conditions are given in Table 1 (denoted as "Product side-draw - 2:1 / 3:1 / 5:1").

As can be seen from Table 1 a selection of the split ratio in the inventive range from 2:1 to 5:1 yields a high-purity n-butanol product. In all cases the energy demand required for operation the dividing wall column is minimized compared to processes known from prior art. Apart from the energy consumption, a stable mode of operation is an important factor for operating the process. One aspect thereof is a sufficient distribution of liquid on the respective zones, for example onto the top of the inflow section and the offtake section. A split ratio from 2:1 to 5:1 has turned out to fulfill all requirements to evenly and efficiently distribute the liquid stream to guarantee a stable operation.

**Table 1**

| **Component** | **Feed** | **Product side-draw** | | |
|---|---|---|---|---|
| | | **2:1** | **3:1** | **5:1** |
| n-butanol | 97.80 | 99.72 | 99.77 | 99.79 |
| C8 and higher | 1.076 | 0.005 | 0.001 | 0.001 |
| 4-heptanone | 0.249 | 0.142 | 0.129 | 0.123 |
| 2-methylbutanol | 0.190 | 0.064 | 0.048 | 0.040 |
| 2-ethylhexanal | 0.289 | 0.020 | 0.008 | 0.005 |
| butyl butyrate | 0.048 | 0.003 | 0.001 | 0.001 |
| butyl acetal | 0.028 | 0.002 | 0.002 | 0.002 |
| isobutanol | 0.061 | 0.014 | 0.013 | 0.012 |
| water | 0.080 | 0.02 | 0.006 | 0.007 |

### Example 2

As a further example, a production-scale dividing wall column according to Fig. 1 was simulated with a state-of-the-art steady-state simulation tool using physical property data that were partly adapted to experimental data.

A crude n-butanol feed with a composition given in Table 2 below (denoted as "Feed") is fed to the inflow section at a flow rate of 30033 kg/h. All compositions in Table 2 are given in percent by weight (wt.-%). The vapor phase at the top of the column is withdrawn and is fed to a total condenser where it is totally condensed except for non-condensable components. The condensed liquid is fed to a phase separator (not shown in Fig. 1) where the organic phase is separated from the aqueous phase. The aqueous phase is withdrawn from the system at a flow rate of 793 kg/h with a composition given in Table 2 (denoted as "Aqueous distillate"). The major part of the organic phase is recycled to the top of the column as reflux at a flow rate of 25912 kg/h. A minor part is withdrawn from the system at a flow rate of 104 kg/h (denoted as "Organic distillate"). A bottom sump stream of 385 kg/h containing high-boiling components is withdrawn from the bottom of the lower section at a temperature of 128.5°C and a composition given in Table 2 (denoted as "Bottoms"). The side-draw containing the desired high-purity n-butanol is withdrawn at a flow rate of 28750 kg/h and a composition given in Table 2 (denoted as "Side draw"). The split ratio between the mass flow rate of the first liquid stream and the mass flow rate of the second liquid stream was 2:1.

**Table 2**

| | **Feed** | **Aqueous distillate** | **Organic distillate** | **Bottoms** | **Side draw** |
|---|---|---|---|---|---|
| Temperature (°C) | 89.5 | 45 | 45 | 128.5 | 122.9 |
| Pressure (bar) | 1.5 | 1 | 1 | 1.2 | 1.2 |
| Flow rate (kg/h) | 30033 | 793 | 104 | 385 | 28750 |
| Water (wt.-%) | 2.5 | 92.7 | 10.9 | 0.0 | 0.0 |
| Lights (wt.-%) | 0.1 | 0.3 | 16.3 | 0.9 | 0.05 |
| n-Butanol (wt.-%) | 96.9 | 7 | 72.8 | 65 | 99.93 |
| Heavies (wt.-%) | 0.5 | 0.0 | 0.0 | 34.1 | 0.02 |

The total amount of energy needed for the separation of n-butanol from the crude n-butanol stream is 8161 kW. The specific amount of energy with respect to the high-purity n-butanol product stream is 8161 kW / 28750 kg/h * 3600 s/h = 1022 kJ/kg. The amount of n-butanol recovered as high-purity product in the side draw corresponds to 98.7 percent by weight of the n-butanol fed to the column.

The distillation system used in this simulation is comparable to the system disclosed in the example of the prior art document EP 2 394 723 B1 (Table 3, Table 4, Figure 7). In that prior art document, a total amount of energy of 5.5 MMKcal/h (corresponding to 6397 kW) is reported. The specific amount of energy with respect to the high-purity n-butanol product stream is 6397 kW / 15208 kg/h * 3600 s/h = 1515 kJ/kg. The amount of n-butanol recovered as high-purity product in the side draw corresponds to 98.7 percent by weight of the n-butanol fed to the column.

Compared to the prior art, a selection of the split ratio in the inventive range from 2:1 to 5:1 yields a high-purity n-butanol product at a comparable recovery rate and a significantly lower specific energy demand required for operating the dividing wall column.

## Claims

1. A method for providing a high-purity n-butanol product stream from a crude n-butanol feed stream by fractional distillation in a dividing wall column (102) comprising an upper section (104), a middle section and a lower section (110),
the middle section comprising an inflow section (106) and an offtake section (108) laterally separated from each other by a dividing wall (112) fixed in the column,
the crude n-butanol feed stream being fed to the inflow section (106) and the high-purity n-butanol product stream being withdrawn from the offtake section (108),
the liquid stream flowing out of the upper section (104) being collected and divided into a first liquid stream (114) and a second liquid stream (116),
the first liquid stream (114) being fed to the upper region of the offtake section (108) and the second liquid stream (116) being fed to the upper region of the inflow section (106), **characterized in that** the crude n-butanol feed stream comprises more than 96 % by weight of n-butanol, less than 2 % by weight of water and less than 2 % of other organic components, the n-butanol product stream contains more than 99 % by weight of n-butanol, a bottom product stream taken off from the bottom of the column contains more than 40 % by weight of n-butanol, and the split ratio between the mass flow rate of the first liquid stream (114) and the mass flow rate of the second liquid stream (116) is from 2:1 to 5:1.

2. The method of claim 1, **characterized in that** the ratio between the cross-sectional area of the inflow section (106) and the cross-sectional area of the offtake section (108) is from 0.9 to 1.1, preferably from 0.95 to 1.05.

3. The method of claim 1 or 2, **characterized in that** the temperature in the lower section (110) is from 115 °C to 130 °C at a pressure from 1.0 to 1.5 bar (abs).

4. The method of any one of claims 1 to 3, **characterized in that** the crude n-butanol feed stream contains more than 300 ppm by weight of butyl butyrate, and the n-butanol product stream contains less than 100 ppm by weight of butyl butyrate.

5. The method of any one of claims 1 to 4, **characterized in that** the flow rates of the first liquid stream (114) and the second liquid stream (116) are measured and that at least one of the two streams is controlled by an actuator to adjust the split ratio to a predefined value between 2:1 and 5:1.

6. The method of any one of claims 1 to 5, **characterized in that** the quality of the n-butanol product stream is controlled to a predefined setpoint by measuring a process value representing the quality in the product stream, in particular the amount of n-butanol in the product stream, as the controlled variable and adjusting the split ratio between the mass flow rates of the first liquid stream (114) and the second liquid stream (116) as the manipulated variable.

## Patentansprüche

1. Verfahren zur Bereitstellung eines hochreinen n-Butanol-Produktstroms aus einem rohen n-Butanol-Zulaufstrom durch fraktionierte Destillation in einer Trennwandkolonne (102), umfassend einen oberen Abschnitt (104), einen mittleren Abschnitt und einen unteren Abschnitt (110),
wobei der mittlere Abschnitt einen Zuführabschnitt (106) und einen Abzugsabschnitt (108) umfasst, die seitlich voneinander durch eine im Kolonneninneren fest angeordnete Trennwand (112) getrennt sind,
wobei der rohe n-Butanol-Zulaufstrom dem Zuführabschnitt (106) zugeführt wird und der hochreine n-Butanol-Produktstrom aus dem Abzugsabschnitt (108) entnommen wird, wobei der aus dem oberen Abschnitt (104) abströmende Flüssigkeitsstrom gesammelt und in einen ersten Flüssigkeitsstrom (114) und einen zweiten Flüssigkeitsstrom (116) aufgeteilt wird,
wobei der erste Flüssigkeitsstrom (114) in den oberen Bereich des Abzugsabschnitts (108) eingespeist wird und der zweite Flüssigkeitsstrom (116) in den oberen Bereich des Zuführabschnitts (106) eingespeist wird,
**dadurch gekennzeichnet, dass** der rohe n-Butanol-Zulaufstrom mehr als 96 Gew.-% n-Butanol, weniger als 2 Gew.-% Wasser und weniger als 2 % andere organische Komponenten umfasst, der n-Butanol-Produktstrom mehr als 99 Gew.-% n-Butanol enthält, ein vom Kolonnenboden abgezogener Bodenproduktstrom mehr als 40 Gew.-% n-Butanol enthält, und das Aufteilungsverhältnis zwischen dem Massenstrom des ersten Flüssigkeitsstroms (114) und dem Massenstrom des zweiten Flüssigkeitsstroms (116) von 2:1 bis 5:1 beträgt.

2. Verfahren nach Anspruch 1, **dadurch gekennzeichnet, dass** das Verhältnis zwischen der Querschnittsfläche des Zuführabschnitts (106) und der Querschnittsfläche des Abzugsabschnitts (108) von 0,9 bis 1,1, vorzugsweise von 0,95 bis 1,05 beträgt.

3. Verfahren nach Anspruch 1 oder 2, **dadurch gekennzeichnet, dass** die Temperatur im unteren Abschnitt (110) von 115 °C bis 130 °C bei einem Druck von 1,0 bis 1,5 bar (abs) liegt.

4. Verfahren nach einem der Ansprüche 1 bis 3, **dadurch gekennzeichnet, dass** der rohe n-Butanol-Zulaufstrom mehr als 300 ppm (Gew.) Butylbutyrat enthält und der n-Butanol-Produktstrom weniger als 100 ppm (Gew.) Butylbutyrat enthält.

5. Verfahren nach einem der Ansprüche 1 bis 4, **dadurch gekennzeichnet, dass** die Durchflussraten des ersten Flüssigkeitsstroms (114) und des zweiten Flüssigkeitsstroms (116) gemessen werden und dass mindestens einer der beiden Ströme durch eine Stelleinrichtung geregelt wird, um das Aufteilungsverhältnis auf einen vorgegebenen Wert zwischen 2:1 und 5:1 einzustellen.

6. Verfahren nach einem der Ansprüche 1 bis 5, **dadurch gekennzeichnet, dass** die Qualität des n-Butanol-Produktstroms auf einen vorgegebenen Sollwert geregelt wird, indem eine die Qualität repräsentierende Prozessgröße, insbesondere der Anteil an n-Butanol im Produktstrom, als Regelgröße gemessen wird und das Aufteilungsverhältnis zwischen den Massenströmen des ersten Flüssigkeitsstroms (114) und des zweiten Flüssigkeitsstroms (116) als Stellgröße eingestellt wird.

## Revendications

1. Procédé pour fournir un flux de produit de n-butanol de haute pureté à partir d'un flux d'alimentation brut en n-butanol par distillation fractionnée dans une colonne à paroi de séparation (102) comprenant une section supérieure (104), une section médiane et une section inférieure (110),
la section médiane comprenant une section d'admission (106) et une section de soutirage (108) latéralement séparées l'une de l'autre par une paroi de séparation (112) fixée dans la colonne,
le flux d'alimentation brut en n-butanol étant alimenté dans la section d'admission (106) et le flux de produit de n-butanol de haute pureté étant soutiré depuis la section de soutirage (108),
le flux liquide s'écoulant de la section supérieure (104) étant collecté et réparti en un premier flux liquide (114) et un second flux liquide (116),
le premier flux liquide (114) étant introduit dans la région supérieure de la section de soutirage (108) et le second flux liquide (116) étant introduit dans la région supérieure de la section d'admission (106),
**caractérisé en ce que** le flux d'alimentation brut en n-butanol comprend plus de 96 % en poids de n-butanol, moins de 2 % en poids d'eau et moins de 2 % d'autres composants organiques, le flux de produit de n-butanol contient plus de 99 % en poids de n-butanol, un flux de produit de fond prélevé au bas de la colonne contient plus de 40 % en poids de n-butanol, et le rapport de répartition entre le débit massique du premier flux liquide (114) et le débit massique du second flux liquide (116) est de 2:1 à 5:1.

2. Procédé selon la revendication 1, **caractérisé en ce que** le rapport entre la surface de section transversale de la section d'admission (106) et la surface de section transversale de la section de soutirage (108) est de 0,9 à 1,1, de préférence de 0,95 à 1,05.

3. Procédé selon la revendication 1 ou 2, **caractérisé en ce que** la température dans la section inférieure (110) est de 115 °C à 130 °C à une pression de 1,0 à 1,5 bar (abs).

4. Procédé selon l'une quelconque des revendications 1 à 3, **caractérisé en ce que** le flux d'alimentation brut en n-butanol contient plus de 300 ppm en poids de butyl butyrate, et le flux de produit de n-butanol contient moins de 100 ppm en poids de butyl butyrate.

5. Procédé selon l'une quelconque des revendications 1 à 4, **caractérisé en ce que** les débits du premier flux liquide (114) et du second flux liquide (116) sont mesurés et qu'au moins l'un des deux flux est régulé par un actionneur afin d'ajuster le rapport de répartition à une valeur prédéfinie comprise entre 2:1 et 5:1.

6. Procédé selon l'une quelconque des revendications 1 à 5, **caractérisé en ce que** la qualité du flux de produit de n-butanol est régulée à une valeur de consigne prédéfinie, en mesurant une grandeur de procédé représentant la qualité dans le flux de produit, en particulier la quantité de n-butanol dans le flux de produit, comme variable contrôlée et en ajustant le rapport de répartition entre les débits massiques du premier flux liquide (114) et du second flux liquide (116) comme variable manipulée.
